(19) Europäisches Patentamt
European Patent Office
Office européen des brevets

(11) Numéro de publication : **0 056 000**
**B1**

(12) **FASCICULE DE BREVET EUROPÉEN**

(45) Date de publication du fascicule du brevet : **26.09.84**

(51) Int. Cl.³ : **C 07 J 41/00**// C07J5/00

(21) Numéro de dépôt : **82200224.2**

(22) Date de dépôt : **23.07.80**

(60) Numéro de publication de la demande initiale en application de l'article 76 CBE : **0023856**

(54) **Dérivés 17/(alkoxycarbonyle) (formamido) méthylène/stéroides et leur préparation.**

(30) Priorité : **31.07.79 FR 7919653**

(43) Date de publication de la demande : **14.07.82 Bulletin 82/28**

(45) Mention de la délivrance du brevet : **26.09.84 Bulletin 84/39**

(84) Etats contractants désignés : **CH DE FR GB IT LI NL SE**

(56) Documents cités :
**CHEMISCHE BERICHTE, vol. 109, no. 12, december 1976 Weinheim, DE U. SCHOLLKOPF et al. : "Notiz zur Synthese von 20-Formamido-3-methoxy-19-nor-1,3,5(10),17(20)-pregnatetraen-21-säure-ethylester aus Ostron-methylether mit Kaliumisocyanessigsäure-ethyl-ester", pages 3964-3965**

(73) Titulaire : **ROUSSEL-UCLAF**
**35, boulevard des Invalides**
**F-75007 Paris (FR)**

(72) Inventeur : **Nedelec, Lucien**
**45, boulevard de l'Ouest**
**F-93340 Le Raincy (FR)**
Inventeur : **Torelli, Vesperto**
**5, rue de la Convention**
**F-94700 Maisons Alfort (FR)**

(74) Mandataire : **Douetteau, Pierre et al**
**c/o ROUSSEL-UCLAF 102, route de Noisy Boite postale 9**
**F-93230 Romainville (FR)**

**EP 0 056 000 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés 17-[(alkoxy carbonyle) (formamido) méthylène] stéroïdes et leur préparation.

L'invention a pour objet les composés de formule (II) :

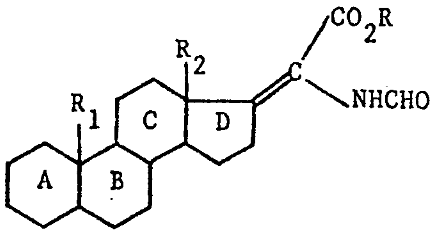

(II)

sous la forme 20E, 20Z ou le mélange 20E et 20Z dans laquelle $R_1$ représente un atome d'hydrogène, un radical alkyle renfermant de 1 à 4 atomes de carbone, éventuellement substitué par une fonction oxygénée ou azotée ou par un atome d'halogène, ou $R_1$ représente un radical alkényle ou alkynyle renfermant de 2 à 4 atomes de carbone, $R_2$ représente un radical alkyle renfermant de 1 à 4 atomes de carbone, les noyaux A, B, C ou D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone, ou par un ou plusieurs radicaux alkényle ou alkynyle renfermant de 2 à 4 atomes de carbone et R représente un radical alkyle renfermant de 1 à 18 atomes de carbone, à l'exception du (20E) 20-formamido 3-méthoxy 19-nor prégna 1,3,5(10)17(20) tétra-èn-21-oate d'éthyle.

Les produits décrits et revendiqués se présentent sous la forme 20E, 20Z ou sous forme d'un mélange. Des exemples de produits 20E ou 20Z ou de mélange 20E et 20Z sont décrits plus loin dans la partie expérimentale.

Lorsque $R_1$ représente un radical alkyle, il s'agit de préférence du radical méthyle ou éthyle.

Lorsque $R_1$ représente un radical alkyle substitué par une fonction oxygénée, il s'agit de préférence du radical hydroxy méthyle ou hydroxy éthyle, du radical formyle ou du radical acétyle.

Lorsque $R_1$ représente un radical alkyle substitué par une fonction azotée, il s'agit de préférence du radical cyano ou du radical aminométhyle ou aminoéthyle.

Lorsque $R_1$ représente un radical alkyle substitué par un halogène, il s'agit de préférence d'un radical $—CH_2Hal$, dans lequel Hal représente un atome d'halogène, comme par exemple un atome de chlore, de fluor ou de brome.

Lorsque $R_1$ représente un radical alkényle, il s'agit de préférence du radical vinyle ou allyle.

Lorsque $R_1$ représente un radical alkynyle, il s'agit de préférence du radical éthynyle.

$R_2$ représente de préférence un radical méthyle ou éthyle.

Lorsque les noyaux A, B, C et D portent une ou plusieurs doubles liaisons, il s'agit de préférence de doubles liaisons en 1(2), 3(4), 4(5) ou 9(11) ou d'un système de doubles liaisons conjuguées en 3(4) et 5(6) ou en 4(5) et 6(7) ou en 1(2) et 4(5) ou d'un système aromatique de trois doubles liaisons 1,3,5 ou d'un système de trois doubles liaisons 1(2), 4(5), 6(7).

Lorsque les cycles A, B, C et D sont substitués par une ou plusieurs fonctions hydroxyles, il s'agit de préférence d'une fonction hydroxyle en 3 ou en 11.

Lorsque les cycles A, B, C et D sont substitués par une ou plusieurs fonctions cétones, il s'agit de préférence d'une fonction cétone en 3 ou en 11.

Lorsque les cycles A, B, C et D sont substitués par une ou plusieurs atomes d'halogènes, il s'agit de préférence d'un atome de fluor, de chlore ou de brome, en position 6 ou 9α par exemple.

Lorsque les cycles A, B, C et D sont substitués par un ou plusieurs radicaux alkyles, il s'agit de préférence du radical méthyle ou éthyle en 2, 6, 7 en 16α ou en 16β.

Lorsque les cycles A, B, C et D sont substitués par un ou plusieurs radicaux alkyloxy, il s'agit de préférence d'un radical méthoxy ou éthoxy en 3 ou 11β.

Lorsque les cycles A, B, C et D sont substitués par un ou plusieurs radicaux alkényles, il s'agit de préférence du radical vinyle ou allyle en position 11β par exemple.

Lorsque les cycles A, B, C et D sont substitués par un ou plusieurs radicaux alkynyles, il s'agit de préférence du radical éthynyle en position 11β par exemple.

L'invention a notamment pour objet les composés de formule (I) pour lesquels $R_2$ représente le radical méthyle et, plus particulièrement, ceux pour lesquels $R_1$ représente un atome d'hydrogène ou un radical méthyle.

L'invention a notamment pour objet les composés de formule (I) répondant à la formule ($II_A$) :

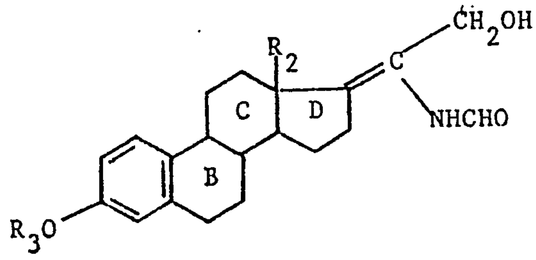

$(II_A)$

dans laquelle $R_2$ représente un radical alkyle renfermant de 1 à 4 atomes de carbone, $R_3$ représente un radical alkyle renfermant de 1 à 8 atomes de carbone et les noyaux B, C et D portent éventuellement une ou plusieurs doubles liaisons et sont éventuellement substitués par un ou plusieurs des substituants définis ci-dessus et R a la signification indiquée ci-dessus.

L'invention a plus particulièrement pour objet les composés de formule $(II_A)$ pour lesquels les cycles B, C et D ne portent aucun substituant.

L'invention a notamment pour objet les composés répondant à la formule $(II_B)$ :

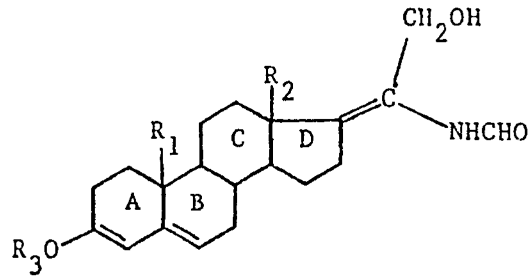

$(II_B)$

dans laquelle $R_1$ et $R_2$ conservent la même signification que ci-dessus, $R_3$ représente un radical alkyle renfermant de 1 à 8 atomes de carbone, les noyaux A, B, C et D portent éventuellement une ou plusieurs doubles liaisons supplémentaires et son éventuellement substitués par un ou plusieurs des substituants définis ci-dessus.

L'invention a plus particulièrement pour objet les composés de formule $(II_B)$ répondant à la formule $(II'_B)$ :

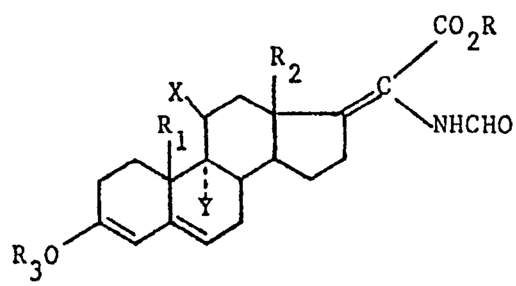

$(II'_B)$

dans laquelle R, $R_1$, $R_2$ et $R_3$ conservent la même signification que précédemment et, ou bien, X et Y représentent chacun un atome d'hydrogène ou bien, X et Y forment ensemble une double liaison carbone-carbone.

Parmi ces composés, l'invention a plus particulièrement pour objet les composés pour lesquels X et Y représentent un atome d'hydrogène ; ceux ayant la configuration 20Z et ceux dans lesquels R représentent un radical éthyle.

L'invention a naturellement tout spécialement pour objet les composés dont la préparation est donnée plus loin dans la partie expérimentale.

Il apparaît clairement à l'homme de métier, notamment à la lecture de la partie expérimentale exposée ci-après, que les composés de l'invention sont d'un très grand intérêt industriel. Ils sont en effet préparés directement avec de très bons rendements à partir des composés 17 cétoniques correspondants par un procédé simple et économique et peuvent être transformés directement en composés portant en 17 la chaîne latérale hydroxyacétyle avec de très bons rendements par un procédé également simple et économique.

La mise en œuvre des composés de l'invention permet donc d'introduire la chaîne hydroxyacétyle à partir de stéroïdes 17-cétoniques par un procédé particulièrement simple, rapide et économique. Il va de soi qu'il s'agit d'un procédé très général applicable à tout stéroïde portant en 17 une fonction cétone. Il a d'ailleurs été indiqué, ci-dessus, que l'invention avait notamment pour objet tous les composés de formule (II) portant en 17 le radical :

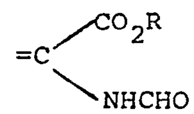

quelle que soit la structure des noyaux A, B, C et D, la nature et le nombre de leurs substituants.

L'invention a également pour objet un procédé de préparation des composés de formule (II), caractérisé en ce que l'on fait agir un composé de formule :

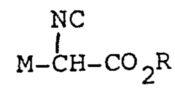

dans laquelle M représente un atome de métal alcalin et R un radical alkyle renfermant de 1 à 18 atomes de carbone, sur un composé de formule :

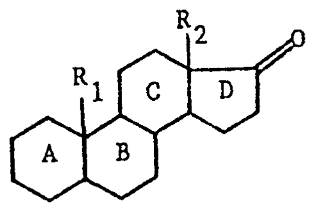

dans laquelle $R_1$ et $R_2$ ont la signification indiquée ci-dessus, les noyaux A, B, C et D portent éventuellement une ou plusieurs doubles liaisons et son éventuellement substitués par un ou plusieurs substituants définis ci-dessus.

Dans un mode de réalisation préféré, M représente un atome de potassium.

Les produits de formule (II) objets de la présente invention peuvent être transformés par action d'un agent de réduction en composés de formule (I) correspondants :

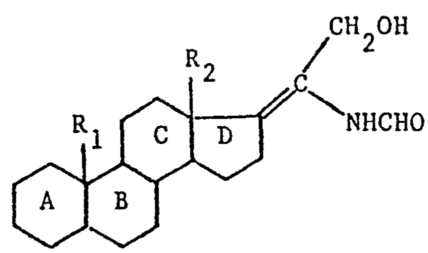

(I)

De préférence, l'agent de réduction est un hydrure d'aluminium, ou bien un dihydro bis alcoxy aluminate de métal alcalin de formule :

$$MAl\ H_2(Oalc_1\ Oalc_2)_2$$

dans laquelle M représente un atome de métal alcalin et $alc_1$ et $alc_2$, identiques ou différents, représentent un radical alkyle renfermant de 1 à 8 atomes de carbone.

Plus préférentiellement, on utilise comme agent de réduction l'hydrure double de lithium et d'aluminium ou le dihydro bis (2-méthoxy éthoxy) aluminate de sodium $Al\ H_2(OCH_2CH_2OCH_3)\ Na$ et l'on opère à une température voisine de 0 °C, par exemple à une température comprise entre − 5 °C et + 5 °C.

Les composés de formule (I) peuvent être soumis à l'action d'un agent d'hydrolyse acide pour obtenir le composé de formule (III) correspondant ;

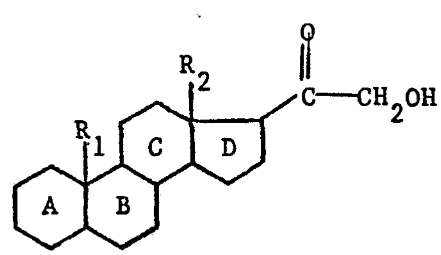

(III)

Il va de soi que l'on peut transformer les composés de formule (II) en composés de formule (III) sans isoler les produits de formule (I).

On utilise de préférence comme agent d'hydrolyse acide, l'acide chlorhydrique ou l'acide sulfurique.

Les composés de formule (II) peuvent donc servir aisément à la préparation de composés d'un très grand intérêt industriel, comme la corticostérone, la 19-nor-corticostérone, la désoxycorticostérone, la 19-nor désoxycorticostérone ou la 9(11) déhydrodésoxycorticostérone qui sont des composés très utiles dans l'industrie pharmaceutique.

Les exemples suivants illustrent l'invention sans toutefois la limiter.

N. B. : On doit remarquer que les produits des exemples 1 à 8 ont été nommés d'après la nomenclature du produit décrit par U. Schollkopf et K. Kantke dans Chem. Ber *109,* 3964 (1976).

Or des travaux menés récemment par la demanderesse ont permis d'établir que la configuration (20E) attribuée par les auteurs au produit préparé est erronée et qu'en fait le produit obtenu possède la configuration (20Z).

Du fait de cette erreur, les produits des exemples 1 à 8 suivants doivent être considérés comme possédant l'isomérie inverse de celle indiquée (les produits dénommés 20E sont en fait 20Z et inversement).

## Exemple 1

(20E) 20-formamido 3-méthoxy 19-nor prégna 1,3,5(10)17(20)-tétraèn 21-ol.

On dissout 1,6 g de (20E) 20-formamido 3-méthoxy 19-nor prégna 1,3,5(10)17(20) tétraèn 21-oate d'éthyle préparé selon le procédé indiqué par U. Schollkopf et K. Hantke Chem. Ber., *109,* 3964 (1976), dans 32 $cm^3$ de tétrahydrofurane anhydre. La solution est agitée à 0 °C pendant deux heures avec 200 mg de borohydrure de potassium et 200 mg d'hydrure double d'aluminium et de lithium. On ajoute ensuite de l'éthanol, goutte à goutte, pour décomposer l'excès d'hydrure double d'aluminium et de lithium, on agite encore une heure à la température ambiante, puis dilue le mélange réactionnel avec une solution de sel de Seignette (tartrate double de potassium et de sodium) et extrait avec l'acétate d'éthyle. On filtre les extraits, les lave à l'eau, les sèche et les distille à sec. On triture le résidu obtenu avec de l'éther isopropylique, glace la suspension, essore, lave à l'éther isopropylique et sèche à l'air.

On obtient 1,28 g du produit recherché que l'on peut utiliser tel que dans le stade suivant (application).

L'échantillon analytique a été obtenu par recristallisation dans le méthanol. F = 191 °C.

Application à la préparation de la 21-hydroxy 3-méthoxy 19-nor prégna 1,3,5(10)-trièn 20-one.

On met en suspension 300 mg du produit brut préparé à l'exemple 1 dans 10 $cm^3$ de méthanol. On ajoute 1 $cm^3$ d'acide chlorhydrique 5N et agite le mélange à la température ordinaire. Après une heure la solution est diluée avec de l'eau. On filtre, lave à l'eau et sèche vers 50 °C le précipité obtenu. On recueille 200 mg de produit recherché.

L'extraction du filtrat avec du chlorure de méthylène fournit encore, après évaporation du solvant 70 mg de produit recherché. Rendement global = 97 %.

L'échantillon analytique fond à 130-131 °C après recristallisation dans du méthanol.

Analyse : $C_{21}H_{28}O_3$ = 328,455

| | | |
|---|---|---|
| Calculé : | C % 76,79 | H % 8,59 |
| Trouvé : | C % 76,6 | H % 8,5 |

Spectre I.R. (chloroforme)

Absence d'OH libre

| | |
|---|---|
| OH associé | 3 458 $cm^{-1}$ |
| 20-céto | 1 706 $cm^{-1}$ |
| aromatique | 1 610, 1 577 et 1 500 $cm^{-1}$ |

Dichroïsme circulaire (éthanol)

Max. 231 nm $\Delta\varepsilon = +2,3$
Max. 287 nm $\Delta\varepsilon = +3,4$

Spectre R.M.N. (chloroforme deutéro)

$H_{20}$ à 4,16-4,25 ppm
H de hydroxyle à 3,17-3,25-3,33 ppm

Exemple 2

(20E) 20-formamido 3-éthoxy prégna 3,5,17(20)-trièn 21-ol.

On dissout 1,025 g d'isomère (20E) 20-formamido 3-éthoxy prégna 3,5,17(20)-trièn 21-oate d'éthyle dans 20 $cm^3$ de tétrahydrofurane anhydre. On agite la solution au bain de glace et ajoute 150 mg d'hydrure double d'aluminium et de lithium, en plusieurs fois, par petites portions. On laisse le mélange réactionnel revenir à la température ordinaire et l'agite pendant 15 heures. On décompose ensuite l'excès d'hydrure par addition, avec précaution, de 5 $cm^3$ d'éthanol. Le mélange réactionnel renferme le dérivé attendu.

Le (20E) 20-formamido 3-éthoxy prégna 3,5,17(20)-trièn 21-oate d'éthyle utilisé au départ de l'exemple a été préparé comme suit :

On dissout 2,15 g de ter-butylate de potassium dans 50 $cm^3$ de tétrahydrofurane anhydre. On agite la solution à − 10 °C et ajoute, en 30 minutes, une solution de 2,25 g d'isocyanacétate d'éthyle dans 20 $cm^3$ de tétrahydrofurane anhydre. Après 15 minutes, on introduit lentement une solution de 5,025 g de 3-éthoxy androsta 3,5-dièn 17-one dans 80 $cm^3$ de tétrahydrofurane anhydre. On laisse ensuite le mélange revenir à température ordinaire et l'agite encore pendant une quinzaine d'heures. Après ce temps, la solution obtenue est versée dans une solution saturée de chlorure d'ammonium et on extrait avec de l'éther éthylique. Les extraits sont lavés à l'eau, séchés et distillés à sec. Le résidu est purifié par chromatographie sur silice (éluant : benzène-acétate d'éthyle 1-1). On sépare dans l'ordre :

— 4,71 g (69 %) d'isomère E du produit recherché.

L'échantillon obtenu par recristallisation dans du méthanol fond à 204 °C.

— 0,97 g (14 %) d'isomère Z, fondant à 172 °C après recristallisation dans du méthanol.

Application à la préparation de la 21-hydroxy prégna 4-ène 3,20-dione.

Le mélange réactionnel obtenu à l'exemple 2 est agité pendant 6 heures à la température ordinaire avec 5 $cm^3$ d'acide chlorhydrique 5N. On dilue ensuite à l'eau et extrait avec de l'acétate d'éthyle. Les extraits sont lavés à l'eau, séchés et distillés à sec. On obtient 600 mg de produit recherché fondant à 141-142 °C après recristallisation dans un mélange de chlorure de méthylène et d'éther isopropylique.

Exemple 3

(20E) 20-formamido 3-méthoxy 19-nor prégna 1,3,5(10)17(20)-tétraèn 21-ol.

A une solution de 1,4 g de (20E) 20-formamido 3-méthoxy 19-nor prégna 1,3,5(10)17(20)-tétraèn 21-oate d'éthyle dans 30 $cm^3$ de tétrahydrofurane anhydre refroidie au bain de glace sous atmosphère d'azote, on ajoute en 10 minutes, 3,5 $cm^3$ de solution benzénique à 70 % de dihydro bis (2-méthoxy éthoxy) aluminate de sodium et agite le mélange pendant une heure. On décompose ensuite l'excès d'hydrure par addition prudente de 10 $cm^3$ d'éthanol, ajoute 0,35 g de borohydrure de potassium et poursuit l'agitation pendant une heure à la température ordinaire.

Le mélange réactionnel renferme le dérivé 21-hydroxy 17(20)-ène 20-formamido attendu.

Application à la préparation de la 21-hydroxy 3-méthoxy 19-nor prégna 1,3,5(10)-trièn 20-one.

On acidifie le milieu réactionnel obtenu à l'exemple 3 avec 20 $cm^3$ d'acide chlorhydrique 5N, agite pendant 15 heures à la température ordinaire. On dilue ensuite à l'eau et extrait avec de l'acétate d'éthyle. Les extraits sont lavés à l'eau, séchés et évaporés à sec.

Le résidu, purifié par chromatographie sur silice (éluant : benzène-acétate d'éthyle 9-1) fournit 0,95 g de produit recherché, sous forme de cristaux incolores fondant à 130-131 °C.

Exemple 4

2,2-diméthyl 3-éthoxy 13β-éthyl 20-formamido 18,19-dinor prégna 3,5,17(20)-trièn 21-ol.

On dissout 850 mg de 2,2-diméthyl 3-éthoxy 13β-éthyl 20-formamido 18,19-dinor prégna 3,5,17(20)-trièn 21-oate d'éthyle dans 8,5 g de tétrahydrofurane, refroidit à 0°, − 2 °C et ajoute 125 mg d'hydrure double d'aluminium et de lithium. Après une heure de réaction à 0 °C, on ajoute avec précaution 8,5 $cm^3$ d'éthanol et 125 mg de borohydrure de sodium. On agite pendant 15 minutes à température ambiante. on dilue le mélange réactionnel avec de l'acétate d'éthyle, ajoute lentement une solution aqueuse, saturée de chlorure de sodium de façon à obtenir une pâte, décante la phase organique et reprend 2 fois le résidu à l'acétate d'éthyle. On lave les phases organiques avec une solution aqueuse saturée de chlorure de sodium, sèche, concentre à sec sous pression réduite et obtient 710 mg de produit attendu (isomères E + Z).

Le 2,2-diméthyl 3-éthoxy 13β-éthyl 20-formamido 18,19-dinor prégna 3,5,17(20)-trièn 21-oate d'éthyle utilisé au départ de l'exemple 4 peut être préparé comme suit :

Stade A

2,2-diméthyl 13β-éthyl 17β-[2' (RS)-tétrahydropyranyloxy)] gona 4-èn 3-one.

Sous agitation et sous atmosphère inerte, on mélange 1,860 g de 13β-éthyl 17β-[2'(RS)-tétrahydropyranyloxy)] gona 4-èn 3-one décrit dans le brevet US 338 928, 10 $cm^3$ de tétrahydrofurane et 4 $cm^3$ d'iodure de méthyle. On refroidit à − 70 °C et introduit goutte à goutte en une heure 20 $cm^3$ d'une solution de terbutylate de potassium 1,4 M dans le tétrahydrofurane et puis agite encore pendant 30 minutes à − 70 °C. On verse le mélange réactionnel dans une solution aqueuse saturée de chlorure d'ammonium et extrait au chlorure de méthylène. Après séchage, puis concentration à sec de la phase organique, on obtient 2,2 g de produit brut attendu.

Stade B

2,2-diméthyl 13β-éthyl 17β-hydroxy gona 4-èn 3-one.

On dissout les 2,2 g de produit obtenu ci-dessus dans 20 $cm^3$ d'éthanol et 4 $cm^3$ HC1 2N. On chauffe au reflux pendant une heure, refroidit, verse dans l'eau et extrait au chlorure de méthylène. On lave la phase organique, sèche et concentre à sec sous pression réduite. On recristallise le résidu dans de l'éther isopropylique et obtient 735 mg de produit. F = 168 °C. Les liqueurs-mères évaporées à sec sont chromatographiées sur silice en éluant par un mélange benzène-acétate d'éthyle (8-2). On recueille 375 mg de produit et le recristallise dans l'éther isopropylique. On obtient 290 mg de produit. F = 169 °C. Après recristallisation du produit chromatographié dans un mélange chlorure de méthylène-éther isopropylique, le produit fond à 170 °C.

Stade C

2,2-diméthyl 13β-éthyl gona 4-ène 3,17-dione.

On refroidit à 0 °C une solution de 2,475 g de produit obtenu précédemment dans 50 $cm^3$ d'acétone, ajoute goutte à goutte 2,6 $cm^3$ de réactif d'Heilbron Jones. On agite encore pendant 15 minutes à 0 °C et ajoute 1 $cm^3$ de méthanol. On dilue à l'eau, essore, lave à l'eau et obtient 2,380 g de produit. F = 195 °C.

Stade D

2,2-diméthyl 3-éthoxy 13β-éthyl gona 3,5-dièn 17-one.

On mélange 1 g de produit obtenu ci-dessus dans 10 $cm^3$ d'éthanol anhydre et 1 $cm^3$ d'orthoformiate d'éthyle. On ajoute 0,25 $cm^3$ de solution à 0,2 % d'acide sulfurique dans l'éthanol et chauffe au reflux sous atmosphère inerte pendant 1 heure 10 minutes. On refroidit, ajoute quelques gouttes de triéthylamine, dilue avec une solution de bicarbonate de sodium et extrait à l'éther. On lave à l'eau, sèche la phase organique et concentre à sec sous pression réduite. On obtient 1,250 g de produit que l'on purifie sur silice en éluant par un mélange benzène-acétate d'éthyle (95-5). On recueille 0,95 g de produit utilisé tel quel pour le stade suivant.

Stade E

2,2-diméthyl 3-éthoxy 13β-éthyl 20-formamido 18-19-dinor prégna 3,5,17(20)-trièn 21-oate d'éthyle, mélange des isomères E et Z.

On mélange 3,36 g de produit obtenu comme ci-dessus dans 15 $cm^3$ de dioxanne et 50 $cm^3$ d'une solution de tert-butylate de potassium 0,93 M dans le dioxanne. On ajoute goutte à goutte, sous atmosphère inerte, 5,25 $cm^3$ d'isocyanoacétate d'éthyle. On chauffe à 80 °C pendant une heure 40 minutes. Après refroidissement, on verse le mélange réactionnel dans une solution aqueuse de phosphate monosodique, extrait à l'acétate d'éthyle, lave à l'eau la phase organique, sèche et concentre à sec sous pression réduite. On chromatographie le résidu sur silice, élue par un mélange benzène-acétate d'éthyle (9-1) puis (7-3) et obtient 3,9 g du produit attendu.

Application à la préparation du 2,2-diméthyl 13β-éthyl 21-hydroxy 18,19-dinor pregn 4-èn 3,20-dione.

On dissout les 710 mg de produit obtenus à l'exemple 4 dans 14 $cm^3$ de méthanol et ajoute 2,8 $cm^3$ d'acide chlorhydrique 2N et laisse à température ambiante pendant 20 heures. On dilue à l'eau, extrait au

chlorure de méthylène, lave la phase organique avec une solution de bicarbonate de sodium, puis à l'eau, sèche, concentre à sec sous pression réduite et obtient 525 mg de produit brut. On le chromatographie sur silice, élue par un mélange benzène-acétate d'éthyle (8-2) et isole 340 mg de produit qui cristallise.

## Exemple 5

3,3-éthylènedioxy 10β-éthynyl 20-formamido 19-nor prégna 9(11)17(20)-dièn 5,21-diol.

On dissout 3,7 g de 3,3-éthylènedioxy 10β-éthynyl 20-formamido 5α-hydroxy 19-nor prégna 9(11) 17(20)-dièn 21-oate d'éthyle dans 90 $cm^3$ de tétrahydrofurane. On refroidit à + 5 °C sous atmosphère inerte et ajoute par fractions, en agitant, 600 mg d'hydrure double d'aluminium et de lithium. Après 2 heures d'agitation, on détruit l'excès d'hydrure par addition lente d'éthanol. On ajoute 30 $cm^3$ d'éthanol et 0,5 g de borohydrure de sodium et agite la suspension pendant 20 minutes à température ambiante. On ajoute 100 $cm^3$ d'acétate d'éthyle et de l'eau contenant du chlorure de sodium, goutte à goutte de façon à précipiter les sels minéraux sous forme de résidu gommeux. On décante la phase organique, reprend le résidu 2 fois à l'acétate d'éthyle. les phases organiques sont séchées, concentrées à sec sous pression réduite. On obtient 2,8 g de produit attendu.

Le 3,3-éthylènedioxy 10β-éthynyl 20-formamido 5α-hydroxy 19-nor prégna 9(11)17(20)-dièn 21-oate d'éthyle utilisé au départ de l'exemple 5 a été préparé comme suit :

### Stade A

3,3-éthylènedioxy 10β-éthynyl estra 9(11)-èn 5α,17β-diol.

On agite 4,12 g de 3,3-éthylènedioxy 5α,10α-époxy 17β-benzoyloxy estra 9(11)-ène (décrit dans le brevet français n° 1 550 974) dans 60 $cm^3$ d'éthylènediamine, ajoute 8,24 g d'acétylure de lithium dans l'éthylène diamine. On chauffe à 45 °C sous atmosphère inerte pendant 24 heures, refroidit dans de la glace et extrait au chlorure de méthylène. on lave à l'eau la phase organique, sèche et concentre à sec. On chromatographie le résidu sur silice, élue par un mélange benzène-acétate d'éthyle (7-3) et isole 3 g de produit qui recristallisé dans l'éthanol aqueux, fond à 213 °C.

### Stade B

3,3-éthylènedioxy 10β-éthynyl 5α-hydroxy estra 9(11)-èn 17-one.

Les 3 g de produit brut obtenus au stade A sont dissous dans 75 $cm^3$ de chlorure de méthylène à 1 % de pyridine. On ajoute 4,5 g de chlorochromate de pyridium Tetrahedron letters n° 31 2647 2650 (1975) et agite pendant une heure à température ambiante. On chromatographie sur silice, élue avec de l'éther et obtient 2,73 g de produit que l'on recristallise dans l'éthanol aqueux, lave, sèche et recueille 2,43 g de produit. F = 188 °C.

### Stade C

3,3-éthylènedioxy 10β-éthynyl 20-formamido 5α-hydroxy 19-nor prégna 9(11)17(20)-dièn 21-oate d'éthyle.

On dissout 2,14 g de 3,3-éthylènedioxy 10β-éthynyl 5α-hydroxy estra 9(11)-èn 17-one dans 21 $cm^3$ de tétrahydrofurane. On ajoute sous agitation et sous atmosphère inerte 10 $cm^3$ de solution de tert-butylate de potassium 1,75 M dans le tétrahydrofurane et 2 $cm^3$ d'isocyanoacétate d'éthyle. Après 1 heure d'agitation à température ambiante, on verse le mélange réactionnel dans l'eau glacée et extrait au chlorure de méthylène. On lave à l'eau la phase organique, sèche et concentre à sec sous pression réduite. on obtient 3,7 g de 3,3-éthylènedioxy 10β-éthynyl 20-formamido 5α-hydroxy 19-nor prégna 9(11) 17(20)-dièn 21-oate d'éthyle.

Application à la préparation de la 10β-éthynyl 21-hydroxy 19-nor prégna 4,9(11)-dièn 3,20-dione.

On dissout 2,68 g du produit obtenu à l'exemple 5 dans 36 $cm^3$ de méthanol contenant 3,6 $cm^3$ d'acide chlorhydrique 6N. On chauffe au reflux pendant 1 heure 20 minutes, refroidit, dilue à l'eau glacée, extrait au chlorure de méthylène, sèche la phase organique et concentre à sec sous pression réduite. On chromatographie le résidu sur silice, élue par un mélange cyclohexane-acétate d'éthyle (7-3). Après recristallisation de différentes fractions dans un mélange chlorure de méthylène-éther isopropylique, on obtient 1,03 g de produit attendu. F = 162 °C.

Spectre RMN ($CDCl_3$)

$CH_3$ en 18 : 0,68 ppm
C≡CH : 2,23 ppm

$CH_2$ en 21 : 4,21 ppm
Hydrogène en 11 : 5,71-5,77-5,81 ppm
Hydrogène en 4 : 5,91 ppm

Exemple 6

(20E) 3-éthoxy 20-formamido 19-nor prégna 3,5,17(20) trièn 21-ol.

On dissout 5,5 g de (20E) 3-éthoxy 20-formamido 19-nor prégna 3,5,17(20-trièn 21-oate d'éthyle dans 100 $cm^3$ de tétrahydrofurane, refroidit à + 5 °C, ajoute 1,2 g d'hydrure double de lithium et d'aluminium et 1 g de borohydrure de sodium et agite pendant 2 heures à + 5 °C. Ensuite, on ajoute, goutte à goutte, 30 $cm^3$ d'éthanol, agite 30 minutes à température ambiante, ajoute 20 $cm^3$ d'une solution de tartrate double de potassium et de sodium et obtient un précipité dans la solution. On décante le surnageant et reprend le précipité à l'acétate d'éthyle plusieurs fois. On réunit les phases organiques, les lave avec une solution aqueuse saturée de chlorure de sodium, sèche et concentre à sec sous pression réduite. On empâte le résidu à l'éther isopropylique, essore et obtient 4,3 g de produit. F = 202 °C.

Spectre UV (éthanol)

Max. 239 nm $E_1^1$ : 668 $\varepsilon$ = 24 800

Le (20E) 3-éthoxy 20-formamido 19-nor prégna 3,5,17(20) trièn 21-oate d'éthyle utilisé au départ de l'exemple 6 a été préparé comme suit :

On dissout 5 g de tert-butylate de potassium dans 60 $cm^3$ de tétrahydrofurane, refroidit à 10 °C, ajoute en 15 minutes, 5 g d'isocyanate d'éthyle dans 30 $cm^3$ de tétrahydrofurane, agite 10 minutes à − 10 °C et ajoute une solution de 9 g de 3-éthoxy estra 3,5-dièn 17-one décrit dans le brevet US 3 029 261 dans 120 $cm^3$ de tétrahydrofurane. On agite le mélange réactionnel pendant 4 heures à température ambiante, verse dans une solution aqueuse saturée de chlorure d'ammonium, extrait à l'acétate d'éthyle, lave à l'eau, sèche, distille à sec sous pression réduite. On cristallise le résidu dans 20 $cm^3$ d'éther et obtient 10,9 g de produit attendu. F = 165 °C.

Spectre UV

dans l'éthanol :
Max. 238 nm $E_1^1$ = 725 $\varepsilon$ = 30 000
dans EtOH HCl N/10
Max. 238 nm $E_1^1$ = 658 $\varepsilon$ = 27 200

Application à la préparation du 21-hydroxy 19-nor pregn 4-èn 3,20-dione.

On dissout 200 mg de produit obtenu à l'exemple 6 dans 5 $cm^3$ de méthanol et 1 $cm^3$ d'acide chlorhydrique 5N et agite une heure à température ambiante. On verse le mélange réactionnel dans une solution aqueuse saturée de bicarbonate de sodium, extrait à l'acétate d'éthyle, sèche et concentre à sec sous pression réduite. On empâte le résidu à l'éther, essore et obtient 160 mg de produit attendu. F = 132 °C.

Par cristallisation dans l'isopropanol, on obtient un produit fondant à 133 °C.

Spectre UV (EtOH)

Max. 240-241 nm $E_1^1$ = 574 $\varepsilon$ = 18 200
Infl. 290 nm $E_1^1$ = 4

Exemple 7

(20E) 3-éthoxy 9α-fluoro 11β-hydroxy 20-formamido prégna 3,5,17(20)-trièn 21-ol.

On dissout 4 g de (20E) 3-éthoxy 9α-fluoro 11β-hydroxy 20-formamido prégna 3,5,17(20)-trièn 21-oate d'éthyle dans 80 $cm^3$ de tétrahydrofurane, refroidit à 0°, + 5 °C, ajoute en 15 minutes par petites fractions, 2 g d'hydrure double de lithium et d'aluminium et maintient encore 1 heure 30 minutes à 0°, + 5 °C sous agitation. On ajoute goutte à goutte 4 $cm^3$ d'une solution aqueuse saturée de chlorure d'ammonium. On essore l'insoluble, lave avec un mélange chloroforme, méthanol (7-3). Les phases organiques sont évaporées à sec. On obtient 2,070 g de produit attendu après chromatographie sur silice du résidu et élution avec un mélange chloroforme-méthanol (95-5) et triéthylamine (0,5°/00).

Spectre UV (EtOH)

Max. 238-239 nm $E_1^1 = 492$ $\varepsilon = 20\,600$

Le (20E) 3-éthoxy 9α-fluoro 11β-hydroxy 20-formamido prégna 3,5,17(20)-trièn 21-oate d'éthyle utilisé au départ de l'exemple 7 a été préparé comme suit :

Sous agitation et sous atmosphère inerte, on ajoute 5,789 g de tert-butylate de potassium à 202 $cm^3$ de tétrahydrofurane, maintenus à 0°, + 5 °C. On ajoute, goutte à goutte, en 15 minutes, une solution de 5,7 $cm^3$ d'isocyanoacétate d'éthyle dans 57 $cm^3$ de tétrahydrofurane et agite encore 15 minutes à 0°, + 5 °C. on introduit alors en 10 minutes une solution de 6 g de 3-éthoxy 9α-fluoro 11β-hydroxy androsta 3,5-dièn 17-one décrit dans le brevet US 3 968 132 dans 120 $cm^3$ de tétrahydrofurane. Après 3 heures 1/4 à température ambiante, on refroidit à + 5° ± 10 °C, ajoute 200 $cm^3$ d'une solution aqueuse saturée de chlorure de sodium et extrait à l'acétate d'éthyle. On lave la phase organique avec une solution saturée de chlorure de sodium, sèche et concentre à sec. On chromatographie le résidu sur silice, élue par un mélange chloroforme-méthanol (95-5) et obtient 5,9 g de produit attendu.

Spectre IR (Ethanol)

Max. 239 nm $E_1^1 = 625$ $\varepsilon = 28\,800$

Application à la préparation de la 11β,21-dihydroxy 9α-fluoro pregn 4-èn 3,20-dione.

On opère de manière analogue à celle décrite dans l'application qui suit l'exemple 6, au départ du produit obtenu -ci-dessus à l'exemple 7 et obtient le produit attendu.

Exemple 8

(20E) 3-éthoxy 11β-hydroxy 20-formamido prégna 3,5,17(20)-trièn 21-ol.

On dissout 10 g de (20E) 3-éthoxy 11-oxo 20-formamido prégna 3,5,17(20)-trièn 21-oate d'éthyle dans 200 $cm^3$ de tétrahydrofurane, refroidit à 0°, + 5 °C pour ajouter 2,266 g de borohydrure de potassium puis, en 10 minutes et par petites fractions, 2,266 g d'hydrure double de lithium et d'aluminium. On agite une heure 50 minutes à 0°, + 5 °C sous atmosphère inerte. On ajoute en 30 minutes et avec précaution, 10 $cm^3$ d'éthanol et agite 30 minutes à 0°, + 5 °C puis 30 minutes en laissant remonter la température. On ajoute 10 $cm^3$ d'une solution aqueuse saturée de chlorure d'ammonium, essore l'insoluble, lave au chloroforme. On concentre à sec le filtrat sous pression réduite et obtient 9 g de produit attendu.

Spectre UV (Ethanol)

Max. 237 nm $E_1^1 = 540$ $\varepsilon = 21\,700$

Le (20E) 3-éthoxy 11-oxo 20-formamido prégna 3,5,17(20)-trièn 21-oate d'éthyle utilisé au départ de l'exemple 8 a été préparé comme suit :

On dissout 16,21 g de tert-butylate de potassium dans 145 $cm^3$ de tétrahydrofurane, refroidit à 0°, + 5 °C, ajoute en 10 minutes sous agitation et sous atmosphère inerte une solution de 15,8 $cm^3$ d'isocyanoacétate d'éthyle dans 95 $cm^3$ de tétrahydrofurane, puis en 20 minutes et à 0°, + 5 °C, 23,8 g de 3-éthoxy 11-oxo androsta 3,5-dièn 17-one décrit dans le brevet US 3 055 917 dans 130 $cm^3$ de tétrahydrofurane. On agite 45 minutes en maintenant la température à 0°, + 5 °C. On ajoute 500 $cm^3$ d'une solution aqueuse saturée de chlorure d'ammonium et extrait à l'acétate d'éthyle. On lave la phase organique avec une solution aqueuse saturée de chlorure d'ammonium, sèche, concentre à sec sous pression réduite. On reprend le résidu par 100 $cm^3$ de chlorure de méthylène, filtre, ajoute 500 $cm^3$ d'acétate d'éthyle, chasse le chlorure de méthylène sous pression réduite, glace, essore le produit cristallisé, lave à l'acétate d'éthyle et obtient 23,7 g de produit attendu. F = 218 °C. Des liqueurs mères chromatographiées sur silice, éluées par un mélange benzène-acétate d'éthyle (7-3), recristallisées dans l'acétate d'éthyle, on recueille 4,814 g de produit. F = 218 °C.

Application à la préparation de la 11β,21-dihydroxy pregn 4-èn 3,20-dione.

On opère de manière analogue à celle décrite dans l'application qui suit l'exemple 6, au départ du produit obtenu ci-dessus à l'exemple 7 et obtient le produit attendu.

**Revendications**

1. Les composés de formule (II) :

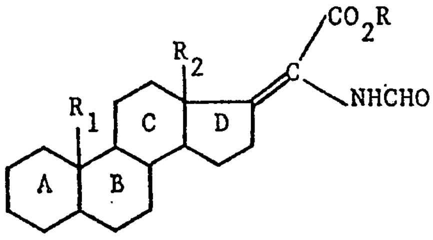

(II)

sous la forme 20E, 20Z ou le mélange 20E et 20Z dans laquelle $R_1$ représente un atome d'hydrogène, un radical alkyle renfermant de 1 à 4 atomes de carbone, éventuellement substitué par une fonction oxygénée ou azotée ou par un atome d'halogène, ou $R_1$ représente un radical alkényle ou alkynyle renfermant de 2 à 4 atomes de carbone, $R_2$ représente un radical alkyle renfermant de 1 à 4 atomes de carbone, les noyaux A, B, C ou D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone, ou par un ou plusieurs radicaux alkényle ou alkynyle renfermant de 2 à 4 atomes de carbone et R représente un radical alkyle renfermant de 1 à 18 atomes de carbone, à l'exception du (20E) 20-formamido 3-méthoxy 19-nor prégna 1,3,5(10)17(20) tétra èn-21-oate d'éthyle.

2. Les composés de formule (II) tels que définis à la revendication 1 dans laquelle $R_1$ représente un atome d'hydrogène ou un radical méthyle et $R_2$ un radical méthyle.

3. Les composés de formule (II) tels que définis à la revendication 1 ou 2, répondant à la formule ($II_A$) :

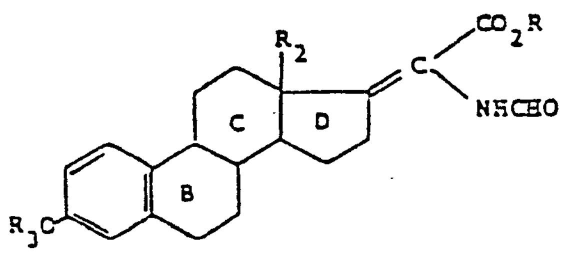

($II_A$)

dans laquelle $R_2$ représente un radical alkyle renfermant de 1 à 4 atomes de carbone, $R_3$ représente un radical alkyle renfermant de 1 à 8 atomes de carbone et les noyaux B, C et D portent éventuellement une ou plusieurs doubles liaisons et sont éventuellement substitués par un ou plusieurs substituants définis à la revendication 1 et R a la signification indiquée à la revendication 1.

4. Les composés de formule ($II_A$) tels que définis à la revendication 3, pour lesquels les cycles B, C et D ne portent aucun substituant.

5. Les composés de formule (II) tels que définis à l'une quelconque des revendications 1 ou 2, répondant à la formule ($II_B$) :

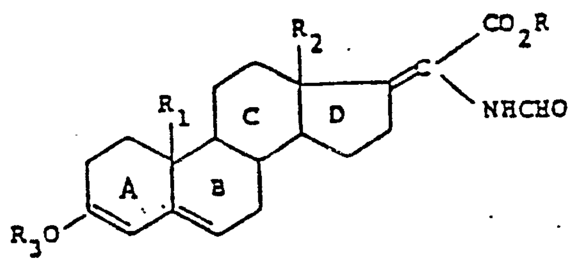

($II_B$)

dans laquelle R, $R_1$ et $R_2$ conservent la même signification que dans la revendication 1, $R_3$ représente un radical alkyle renfermant de 1 à 8 atomes de carbone, les noyaux A, B, C et D portent éventuellement une ou plusieurs doubles liaisons supplémentaires et sont éventuellement substitués par un ou plusieurs des substituants définis à la revendication 1.

6. Les composés de formule ($II_B$) tels que définis à la revendication 5, répondant à la formule ($II'_B$) :

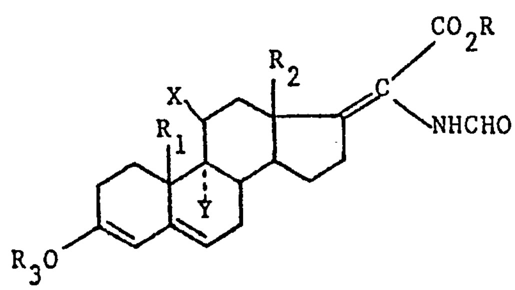

($II'_B$)

dans laquelle R, $R_1$, $R_2$ et $R_3$ conservent la même signification que dans la revendication 5 et, ou bien, X et Y représentent chacun un atome d'hydrogène ou bien, X et Y forment ensemble une double liaison carbone-carbone.

7. Les composés de formule ($II'_B$) tels que définis à la revendication 6, pour lesquels X et Y représentent un atome d'hydrogène.

8. Les composés de formule (II) tels que définis à l'une quelconque des revendications 1 à 7 ayant la configuration 20Z.

9. Les composés de formule (II) tels que définis à l'une quelconque des revendications 1 à 8 dans lesquels le substituant R représente un radical éthyle.

10. Procédé de préparation des composés de formule (II) tels que définis à la revendication 1, caractérisé en ce que l'on fait agir un composé de formule :

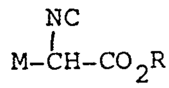

dans laquelle M représente un atome de métal alcalin et R un radical alkyle renfermant de 1 à 18 atomes de carbone, sur un composé de formule :

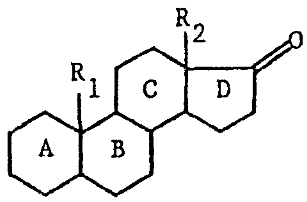

dans laquelle $R_1$ et $R_2$ ont la signification indiquée à la revendication 1, les noyaux A, B, C et D portent éventuellement une ou plusieurs double liaisons et sont éventuellement substitués par un ou plusieurs substituants définis à la revendication 1.

**Claims**

1. The compounds with the formula (II) :

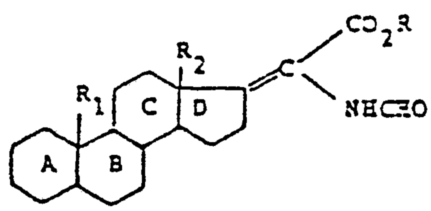 (II)

in the form of 20E, 20Z or a mixture of 20E and 20Z in which $R_1$ represents a hydrogen atom, an alkyl radical containing 1-4 carbon atoms, possibly substituted by an oxidized or nitrogenated function or by a halogen atom, or $R_1$ represents an alkenyl or alkynyl radical containing 2-4 carbon atoms, $R_2$ represents an alkyl radical containing 1-4 carbon atoms, the nuclei A, B, C or D possibly carry one or more double bonds and are possibly substituted by one or more hydroxyl or ketone functions, by one or more halogen atoms, by one or more alkyl or alkyloxy radicals containing 1-4 carbon atoms, or by one or more alkenyl or alkynyl radicals containing 2-4 carbon atoms and R represents an alkyl radical containing 1-18 carbon atoms, with the exception of (20E)-20-formamido-3-methoxy-19-norpregna-1,3,5(10)17(20)-tetraen-21-oate of ethyl.

2. The compounds with the formula (II) as defined in Claim 1, in which $R_1$ represents a hydrogen atom or a methyl radical and $R_2$ represents a methyl radical.

3. The compounds with the formula (II) as defined in Claim 1 or 2, corresponding to the formula ($II_A$) :

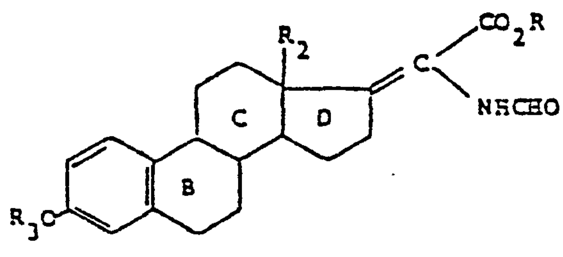 ($II_A$)

in which $R_2$ represents an alkyl radical containing 1-4 carbon atoms, $R_3$ represents an alkyl radical containing 1-8 carbon atoms and the nuclei B, C and D possibly carry one or more double bonds and are possibly substituted by one or more substituents as defined in Claim 1 and R has the significance indicated in Claim 1.

4. The compounds with the formula ($II_A$) as defined in Claim 3, for which the rings B, C and D carry no substituents.

5. The compounds with the formula (II) as defined in any one of Claims 1 or 2, corresponding to the formula ($II_B$) :

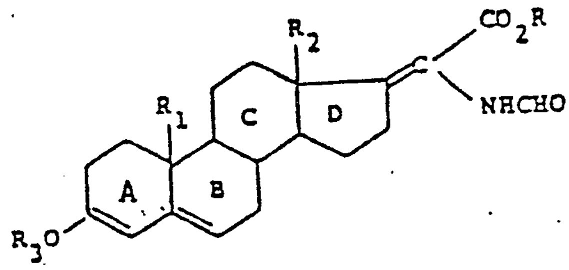

($II_B$)

in which R, $R_1$ and $R_2$ retain the same significance as in Claim 1, $R_3$ represents an alkyl radical containing 1-8 carbon atoms, the nuclei A, B, C and D possibly carry one or more additional double bonds and are possibly substituted by one or more substituents as defined in Claim 1.

6. The compounds with the formula ($II_B$) : as defined in Claim 5, corresponding to the formula ($II'_B$) :

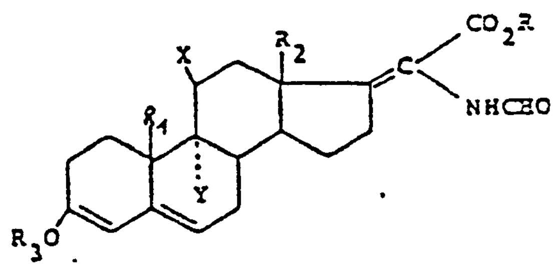

($II'_B$)

in which R, $R_1$, $R_2$ and $R_3$ retain the same significance as in Claim 5 and, either X and Y each represent a hydrogen atom, or X and Y together form a carbon-carbon double bond.

7. The compounds with the formula ($II'_B$) as defined in Claim 6, for which X and Y represent a hydrogen atom.

8. The compounds with the formula (II) as defined in any one of Claims 1-7, with 20Z configuration.

9. The compounds with the formula (II) as defined in any one of Claims 1-8, in which the substituent R represents an ethyl radical.

10. Preparation process for the compound with the formula (II) as defined in Claim 1, characterized in that a compound with the formula :

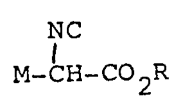

in which M represents an alkali metal atom and R represents an alkyl radical containing 1-18 carbon atom, is made to act on a compound with the formula :

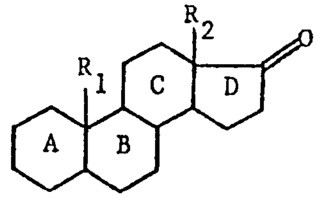

in which $R_1$ and $R_2$ have the significance indicated in Claim 1, the nuclei A, B, C and D possible carry one or more double bonds and are possibly substituted by one or more substituents as defined in Claim 1.

**Ansprüche**

1. Verbindungen der Formel (II)

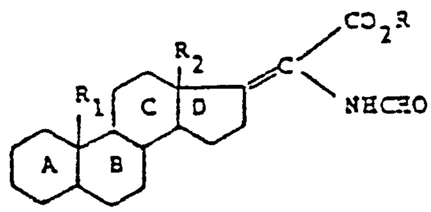

(II)

in der 20E- oder 20Z-Form oder als Gemisch der 20E- und 20Z-Form, worin $R_1$ ein Wasserstoffatom, einen Alkylrest mit 1 bis 4 Kohlenstoffatomen, der gegebenenfalls durch eine Sauerstoff oder Stickstoff enthaltende Funktion oder durch ein Halogenatom substituiert ist, bedeutet oder $R_1$ einen Alkenyl- oder Alkinylrest mit 2 bis 4 Kohlenstoffatomen darstellt, $R_2$ einen Alkylrest mit 1 bis 4 Kohlenstoffatomen bedeutet, die Ringe A, B, C oder D gegebenenfalls eine oder mehrere Doppelbindungen enthalten und gegebenenfalls substituiert sind durch ein oder mehrere Hydroxyl- oder Ketonfunktionen, durch ein oder mehrere Halogenatome, durch ein oder mehrere Alkyl- oder Alkoxyreste mit 1 bis 4 Kohlenstoffatomen, oder durch ein oder mehrere Alkenyl- oder Alkinylreste mit 2 bis 4 Kohlenstoffatomen, und R einen Alkylrest mit 1 bis 18 Kohlenstoffatomen bedeutet, ausgenommen das Äthyl-(20E)-20-formamido-3-methoxy-19-nor-pregna-1,3,5(10),17(20)-tetraen-21-oat.

2. Verbindungen der Formel (II) gemäß Anspruch 1, worin $R_1$ ein Wasserstoffatom oder einen Methylrest bedeutet und $R_2$ ein Methylrest bedeutet.

3. Verbindungen der Formel (II) gemäß Anspruch 1 oder 2, der Formel ($II_A$)

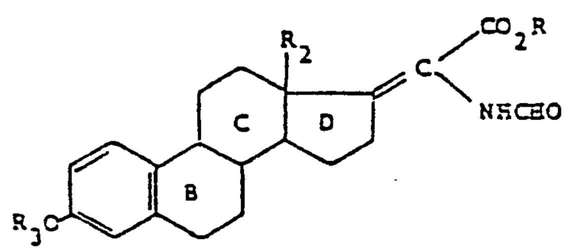

($II_A$)

worin $R_2$ einen Alkylrest mit 1 bis 4 Kohlenstoffatomen bedeutet, $R_3$ einen Alkylrest mit 1 bis 8 Kohlenstoffatomen bedeutet und die Ringe B, C und D gegebenenfalls ein oder mehrere Doppelbindungen enthalten und gegebenenfalls substituiert sind durch ein oder mehrere der in Anspruch 1 definierten Substituenten und R die in Anspruch 1 angegebene Bedeutung besitzt.

4. Verbindungen der Formel ($II_A$) gemäß Anspruch 3, worin die Ringe B, C und D keinen Substituenten enthalten.

5. Verbindungen der Formel (II) gemäß einem der Ansprüche 1 oder 2, der Formel ($II_B$)

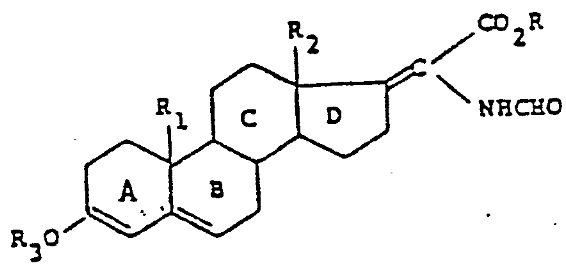

($II_B$)

worin R, $R_1$ und $R_2$ die in Anspruch 1 angegebene Bedeutung besitzen, $R_3$ einen Alkylrest mit 1 bis 8 Kohlenstoffatomen bedeutet, die Ringe A, B, C und D gegebenenfalls ein oder mehrere zusätzliche Doppelbindungen enthalten und gegebenenfalls substituiert sind durch ein oder mehrere der in Anspruch 1 definierten Substituenten.

6. Verbindungen der Formel ($II_B$) gemäß Anspruch 5, der Formel ($II'_B$)

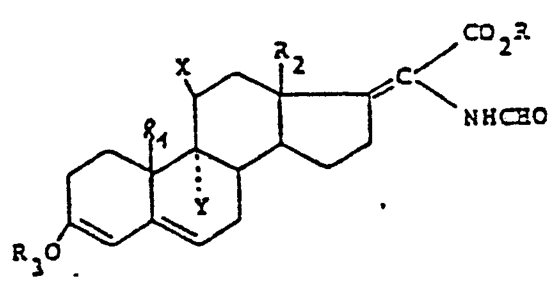

($II'_B$)

worin R, $R_1$, $R_2$ und $R_3$ die in Anspruch 5 angegebene Bedeutung besitzen und entweder X und Y jeweils ein Wasserstoffatom bedeuten oder X und Y gemeinsam eine Kohlenstoff-Kohlenstoffdoppelbindung bilden.

7. Verbindungen der Formel ($II'_B$) gemäß Anspruch 6, worin X und Y ein Wasserstoffatom bedeuten.

8. Verbindungen der Formel (II) gemäß einem der Ansprüche 1 bis 7, mit der 20Z-Konfiguration.

9. Verbindungen der Formel (II) gemäß einem der Ansprüche 1 bis 8, worin der Substituent R einen Äthylrest bedeutet.

10. Verfahren zur Herstellung der Verbindungen der Formel (II) gemäß Anspruch 1, dadurch gekennzeichnet, daß man eine Verbindung der Formel

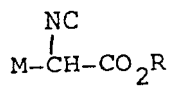

worin M ein Alkalimetallatom bedeutet und R einen Alkylrest mit 1 bis 18 Kohlenstoffatomen bedeutet, mit einer Verbindung der Formel

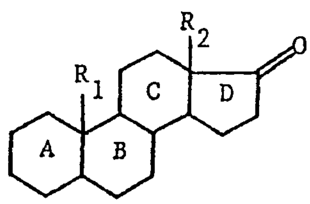

worin $R_1$ und $R_2$ die in Anspruch 1 angegebene Bedeutung besitzen, die Ringe A, B, C und D gegebenenfalls ein oder mehrere Doppelbindungen enthalten und gegebenenfalls durch ein oder mehrere der in Anspruch 1 definierten Substituenten substituiert sind, umsetzt.